# EUROPEAN PATENT SPECIFICATION

(11) **EP 3 473 236 B1**
(45) Date of publication and mention of the grant of the patent: **28.04.2021**
(21) Application number: 17196940.5
(22) Date of filing: 17.10.2017
(51) Int. Cl.: A61K 8/34, A61K 8/368, A61Q 1/00, A61K 8/73, A61Q 17/04, A61Q 19/00, A61K 8/81, A61K 8/02, A61K 8/04

(54) **MICROGELS FOR THE DELIVERY OF COSMETIC ACTIVE ORGANIC SUBSTANCES**
MIKROGELE ZUR ABGABE KOSMETISCHER ORGANISCHER WIRKSTOFFE
MICROGELS POUR ADMINISTRATION DE SUBSTANCES ORGANIQUES ACTIVES COSMÉTIQUES

(43) Date of publication of application: 24.04.2019
(73) Proprietor: LvmH Recherche, 45800 Saint-Jean De Braye (FR); CENTRE NATIONAL DE LA RECHERCHE SCIENTIFIQUE, 75016 Paris (FR); UNIVERSITE DE PAU ET DES PAYS DE L'ADOUR, 64000 Pau (FR)
(72) Inventor: Alard, Valérie, 45000 Orléans (FR); Aguirre, Garbine, 64000 Pau (FR); Billon, Laurent, 64110 Saint Faust (FR)
(74) Representative: Cabinet Beau de Loménie

(56) References cited:
- WO-A1-2016/110615
- WEITAI WU ET AL: "Multi-functional core-shell hybrid nanogels for pH-dependent magnetic manipulation, fluorescent pH-sensing, and drug delivery", BIOMATERIALS, ELSEVIER SCIENCE PUBLISHERS BV., BARKING, GB, vol. 32, no. 36, 31 August 2011 (2011-08-31), pages 9876-9887, XP028316441, ISSN: 0142-9612, DOI: 10.1016/J.BIOMATERIALS.2011.08.082 [retrieved on 2011-09-02]
- Anonymous: "Fluorouracil - Wikipedia", , 16 January 2018 (2018-01-16), XP055442272, Retrieved from the Internet: URL:https://en.wikipedia.org/wiki/Fluorour acil [retrieved on 2018-01-18]
- ESTHER CAZARES-CORTES ET AL: "Doxorubicin Intracellular Remote Release from Biocompatible Oligo(ethylene glycol) Methyl Ether Methacrylate-Based Magnetic Nanogels Triggered by Magnetic Hyperthermia", ACS APPLIED MATERIALS & INTERFACES, vol. 9, no. 31, 27 July 2017 (2017-07-27), pages 25775-25788, XP055442230, US ISSN: 1944-8244, DOI: 10.1021/acsami.7b06553

## Description

The present invention deals with organic cosmetic active molecules encapsulation and controlled release. Hydrophilic and hydrophobic molecules are trapped into temperature-responsive and pH-responsive oligo(ethylene glycol)-based biocompatible microgels which can then be incorporated into cosmetic compositions.

### PRIOR ART

Many macromolecular systems have been developed as delivery systems for biological active molecules in the body via intravenous route.

Among them, aqueous colloidal dispersions of cross-linked polymers called microgels are solvent-swollen networks having recently been growing in popularity due to their potential as a method of specific tissue targeting drug delivery. They can release the active substance via a collapse-to-swelling mechanism that is usually triggered by a pH or a temperature variation.

The main temperature-responsive microgels are based on poly(N-isopropylacrylamide) (PNIPAM), and less commonly on poly(N-vinylcaprolactam) (PVCL) or on poly(oligo(ethylene glycol)methacrylate.

For example, Peng et al. - in Nanoscale, 2012, 4, 2694-2704 - produced pH-ionizable poly(N-isopropylacrylamide)-poly(methacrylic acid) microgels for the delivery of bovine serum albumin (BSA) as a hydrophilic molecule. Anti-cancer drugs such as doxorubicine have been encapsulated in another type of core-shell microgels comprising a poly(2-(dimethylamino)ethyl methacrylate) core and a poly(N-vinylcapronactam) microgels shell (Guarbine et al., Journal of Polymer Science, 2016, 54, 1694-1705*.* However, encapsulated amount of drugs in these microgels was low, and poly(N-isopropylacrylamide) are not biocompatible.

Other temperature-responsive microgels are based on poly(oligo(ethylene glycol)methacrylates core-shell particles comprising a hydrophobic core of poly(di(ethylene glycol) methyl ether methacrylate) and a hydrophilic shell consisting of a copolymer of di(ethylene glycol) methyl ether methacrylate) and penta(ethyleneglycol) methyl ether methacrylate. These microgels have been tested for the delivery of a hydrophobic model drug (dipyridamole) (Zhou et al., Polymer, 2010, 51, p. 3926-3933). The core-shell structure is however complex to obtain and requires use of a surfactant. Release of the active molecule is only 14% at pH 7.4 after 28 h.

Oligo(ethylene glycol) ortho esters that are crosslinked with breakable disulfide bonds have been also proposed (Qiao, Z. et al., Journal of Controlled Release, 2011, 152, 57-66*).* Furthermore, synthesis of hyaluronic acid-based microgels has been reported in C. Luo, J. Zhao, M. Tu, R. Zeng, J. Rong, Mat Sci. Eng. C., 2014, 36, 301. However, encapsulated amounts of active molecules in these microgels were very low.

All the above-reported microgels fail the disadvantage of not being pH-responsive and cannot serve as a versatile and tunable efficient delivery system. That is why temperature-responsive and pH-responsive microgels that are based on oligo(ethylene glycol)-methacrylate-acrylic acid have been since then proposed for pharmaceutical delivery applications.

Cai (Nanotech. Biol. And Med. 8, 2012*)* disclosed microgels comprising a mixture of two polymers that are not covalently bonded: a first network of a crosslinked copolymer of tetra(ethylene glycol) methyl ether methacrylate and di(ethylene glycol) ethyl ether methacrylate, and a second network of a 137.000 molecular weight polyacrylic acid for the encapsulation of the bovine serum albumin protein. In WO 2015/027342, a crosslinked copolymer of di(ethylene glycol) ethyl ether methacrylate and acrylic acid having disulfide links is used for the delivery of 3(R)-[(2(S)-pyrrolidmylcarbonyl)amino]-2-oxo-pyrrolidineacetamide into the brain. However, encapsulation amounts of active molecules are very low in both systems: the amounts are 10 microgram/mg and 39 microgram/mg respectively.

Weitai Wu et al. in "Multi-functional core-shell hybrid nanogels for pH-dependent magnetic manipulation, Fluorescent pH-sensing and drug delivery, Biomaterials, Vol. 32 N°36, 2011, 9876-9887" have prepared nanogels starting from Ni-Ag nanoparticule core and covering them with a shell polymer made of di(ethylene glycol) methyl ether methacrylate, oligo(ethylene glycol) methyl ether methacrylate, methacrylic acid and poly(ethyleneglycol) dimethacrylate.

Esther Cazares-Cortes et al. « Dororubicine Applied Materials Doxorubicin Intracellular Remote Release from Biocompatible Oligo(ethylene glycol) Methyl Ether Methacrylate-Based Magnetic Nanogels Triggered by Magnetic Hyperthermia", Appl. Mater. Interfaces 2017, 9, 31, 25775-25788 have prepared nanogels comprising ferromagnetic nanoparticles and dororubicine. The nanogels are prepared from MEO2MA, OEGMA, MAA and OEGMA (Mn=250g/mol).

WO 2016/110615 A1 teaches the loading of a crosslinked poly(ethylene glycol) methyl ether methacrylate polymer with a ferrofluid, the weight ratio between the ferrofluid and the bare microgels being up to 33% in the loaded microgel (which is equivalent to 330 microgram/mg).

All these past developed thermo-responsive microgels suffer the disadvantage of containing a limited amount of encapsulated organic molecules, and the disadvantage of not releasing a significant amount of encapsulated drug. Moreover, they have been specifically designed for intravenous administration.

Therefore, there is a need for biocompatible microgels that can simultaneously contain a high encapsulated amount of organic molecules, maintain colloidal stability and provide intense collapse-to-swelling behavior, all these three concomitant conditions being necessary for a quantitative release of drug.

There is a need for biocompatible microgels, specifically adapted to skin delivery of cosmetic active organic molecules, which can be effective when put into contact with the skin surface that has a particular temperature and a particular pH. Indeed, intravenous administration conditions and topical administration conditions are so different that adapted microgel chemistry to the first is not adapted to the second.

There is a further need to provide microgels that can be incorporated into conventional cosmetic compositions that have specific pH and specific ionic strength values, in a stable manner.

There is still another need for temperature-responsive and pH-responsive biocompatible microgels that can encapsulate high amounts of organic molecules with satisfactory encapsulation efficiency and appropriate time release kinetics.

Forming stable self-assembled microgel films is not easy because microgel particles having a high colloidal stability in water due to the electrostatic repulsive forces of their electrical double layer and a highly hydrophilic behavior are needed. Early research in this direction has been mainly focused on the creation of electrostatic or covalent interactions between particles in order to stabilize self-assembled microgel films. In these works, cross-linking of microgel particles was promoted by external triggers and long casting periods. These methods and chemistries are not appropriate for cosmetic applications for which spontaneous film formation on skin is required.

Therefore, a need exists to provide biocompatible microgels films that can form on skin through a simple water evaporation mechanism, that can encapsulate significant amounts of organic molecules and release organic molecules at efficient doses.

### DEFINITIONS

A "hydrophilic molecule" can engage hydrogen bonding with water or a polar solvent. It can dissolve easier in water than in oils or other hydrophobic solvents. For example, a hydrophilic molecule can have a solubility in water at 25°C that is higher than 0.1 g/L. A "hydrophobic molecule" is a molecule that is not a hydrophilic molecule.

The term "entrap" means that the organic molecule is located within the polymer network of the microgel. The network of the crosslinked polymer can form a barrier around the molecule that can be suppressed by some physical change in the network, for example with a pH variation trigger, a temperature variation trigger, or a solvent variation trigger. The entrapped organic molecule is preferably not linked to the crosslinked polymer with a covalent bond. The entrapped organic molecule can have electrostatic interactions, Van der Walls bonds or hydrogen bonds with the crosslinked polymer, that can be engaged between C=C bonds of -OH groups of the organic molecules and ethylene glycol moieties of the crosslinked polymer.

The term "delivery of an active molecule or substance" encompasses an immediate release, a sustained release, a controlled release, an extended release and/or a targeted release of the cosmetic active organic substance. The targeted-release can make the active molecule reach and interact with a biological target within the body to cause a physiological effect that would be lessened in the absence of microgel encapsulation.

Within the meaning of the invention a "unloaded microgel particles" is understood to be a crosslinked polymer in the form of a spherical particles having a size that varies from 100 nm to 500 nm in the dry state (i.e. containing less than 2% by weight of water), preferably between 350 and 450 nm, more preferably of the order of 400 nm. The microgel of the invention can be obtained by an aqueous phase copolymerization of several monomers.

"Microgels", in the sense of the present description, is in the form of an aqueous dispersion of unloaded microgel particles or in the form of a film comprising unloaded microgel particles, wherein the unloaded microgel particles are defined as above, and wherein the unloaded microgel particles entrap cosmetic active organic molecules. Microgels can also be named "loaded microgels" or "loaded microgel particles" in the present description. The film can have a thickness being from 10 to 500 microns or from 100 to 400 microns.

In a particular embodiment, the unloaded microgel particles that form a part of microgels of the invention preferably do not have a core part and a shell part, wherein each part have different monomer compositions. The microgels of the invention preferably comprise only one type of a crosslinked copolymer.

Crosslinking density of the copolymer in the microgels may vary within the particle volume generating thereby a "core-shell" structure comprising two parts: one of the two parts having a crosslinking density that is lower than the other part.

The "amount of the cosmetic active organic substance in the loaded microgels" is the weight (in microgram symbol "µg") of the cosmetic active organic substance that is entrapped in the crosslinked polymer per 1 mg of crosslinked polymer in the loaded microgels. The "amount of the cosmetic active organic substances in the loaded microgels" is also mentioned as "the entrapped substance amount" in the rest of the description.

The "amount of the cosmetic active organic substance in the feeding solution" - also called "the feeding substance amount" in the following description - is the weight of the cosmetic active organic substance in the feeding solution (in µg or mg) per 1 mg of unloaded microgel particles that are used to entrap the active substance. The feeding substance amount unit may be written in a shorter way "mg/mg" or "microgram/mg".

The Entrapment efficiency (EE%) is defined as the ratio of the weight of the cosmetic active organic substance that is entrapped in the loaded microgels and the amount of the cosmetic active organic substance that is contained in the feeding solution. The Entrapment efficiency (EE%) can also be defined as the ratio A/B of the entrapped substance amount (A) and the feeding substance amount (B), as defined here above.

A "crosslink" is a moiety (part of a molecule) that links the copolymer chains together. This crosslink derives from a "crosslinker" molecule that is mixed with the monomers during the polymerization process of the crosslinked polymer.

In the sense of the invention, an "organic substance" is a compound that contains carbon and hydrogen atoms. An organic substance is not a ferrofluid.

### DESCRIPTION OF THE INVENTION

The invention relates to microgels for the delivery of a cosmetic active organic substance, which substance is entrapped in a at least one crosslinked poly(ethylene glycol) methyl ether methacrylate polymer, wherein the weight ratio of cosmetically active organic substance to crosslinked polymer in the microgel is from 500 microgram/mg to 10 mg/mg and wherein the crosslinked polymer comprises copolymer chains having diethylene glycol methacrylate monomeric units, oligoethylene glycol methacrylate monomeric units comprising from 6 to 10 ethylene glycol moieties, methacrylic acid monomeric units, said copolymer chains being linked with crosslinks each having di(meth)acrylate end groups and a moiety selected in the group consisting of -(CH2-CH2-O)n-CH2-CH2- where n is from 0 to 6 and preferably from 4 to 5, -NH-CH2-NH- and mixtures thereof..

Microgels comprising a crosslinked poly(ethylene glycol) methyl ether methacrylate polymer entrapping magnetic inorganic nanoparticles have been already proposed by M. Boularas et al. in Polym. Chem., 7, (2016), 350-363*.* The encapsulation of inorganic ferrofluids is made effective through electrostatic interactions and no release of the particles is possible, which is not the case of the microgels of the invention.

The crosslinked polymer can have a constant crosslinking rate within the entire volume of the microgel particle. On the contrary, crosslinking density can be higher or lower at the surface of the particles.

It is preferred that microgel particles consist of organic compounds. For example, microgel particles do not contain any silica, especially silica as a support for the crosslinked polymer.

Encapsulated amount of active molecules can proportionally increase with the feeding active molecule solution that is used for encapsulation process, and an unexpected high value of 750 µg/(mg of unloaded microgel), and even 1,000 µg/(mg of unloaded microgel) can be achieved, in the case of hydrophilic as well as in the case of hydrophobic active substances. It has been observed that the encapsulated amount was independent of encapsulation temperature, at least in the tested concentrations, i.e. the encapsulated amounts were the same being the microgel particles swollen or collapsed.

Synthesized microgels of the invention may have thermo-responsive swelling-collapse transition in buffered media having different ionic strengths. They advantageously show responsiveness in cosmetic media and can therefore be used as delivery systems for cosmetic active substances. The inventors have surprisingly found that average hydrodynamic particle diameter of the microgels varies depending on pH, temperature and/or hydrophobicity of different buffered media that mimic cosmetic ones.

The microgels of the invention comprise a mixture of branched ethylene oxide monomeric units and monomeric units comprising a carboxylic acid or carboxylate group.

Multi-responsiveness in buffered media of microgels of the invention combined with high encapsulating amounts and high releasing amounts of cosmetic active molecules that can both be performed in a controlled way offer a very advantageous dermal delivery system.

Controllable release of organic cosmetic molecules from a cosmetic product into skin and targeted delivery in specific skin areas are necessary to observe a biological effect and to guarantee an optimal biological performance. Delivery of active molecules at a biological target is necessary for the projected biological effect.

According to one embodiment, the microgels of the invention can be obtained by aqueous phase precipitation polymerization of the following three monomers:
- di(ethylene glycol) methyl ether methacrylate,
- an oligo(ethylene glycol) methyl ether methacrylate comprising from 6 to 10 ethylene glycol moities, more preferably from 7 to 9 ethylene glycol moities, and most preferably from 8 to 9 ethylene glycol moieties,
- a (meth)acrylic acid monomer,
in the presence of the crosslinking agent.

In the initial monomer mixture, di(ethylene glycol) methyl ether methacrylate represents for example 50 mol% to 90 mol% of the total number of moles of the monomers, oligo(ethylene glycol) methyl ether methacrylate preferably represents 5 to 50 mol% of the total number of moles of the monomers and the (meth)acrylic acid monomer preferably represents 0.1 mol% to 20 mol%, for example ranging from 0.1 to 5 mol%, of the total number of moles of the monomers, the sum of these three contents being equal to 100%.

The molar ratio (a:b) between di(ethylene glycol) methyl ether methacrylate (a) and oligo(ethylene glycol) methyl ether methacrylate (b) is preferably between 1:1 and 20:1, for example between 5:1 and 10:1.

Within the meaning of the invention the expression "between" excludes the numerical limits that succeed it. On the other hand, the expression "ranging from ... to" includes the stated limits.

The monomer di(ethylene glycol) methyl ether methacrylate that is used to prepare the crosslinked polymer of the invention represents for example 80 to 90 mol% of the total number of moles of the three monomers, the oligo(ethylene glycol) methyl ether methacrylate monomer preferably represents 5 to 15 mol% of the total number of moles of the monomers and methacrylic acid preferably represents 0.1 to 10 mol% of the total number of moles of the monomers, the sum of these three contents being equal to 100%.

The (meth)acrylic acid monomer can have the formula CR₁R₂=CR₃R₄ in which R₁, R₂, R₃ and R₄ represent a hydrogen, a halogen or a hydrocarbon group, at least one of the four groups comprising a -COOH or -COO-M+ group, M+ representing a cation.

The (meth)acrylic acid monomer can be selected from the group consisting of methyl acrylic, methyl methacrylic, ethyl acrylic, ethyl methacrylic, n-butyl acrylic, and n-butyl methacrylic, methacrylic, itaconic or acrylic acids. Methacrylic acid is preferred.

In a particular embodiment, the molar fraction of diethylene glycol methacrylate monomeric units is from 80 mol.% to 90 mol.%, preferably from 82 mol.% to 86 mol.%, the molar fraction of oligoethylene glycol methacrylate monomeric units is from 5 mol.% to 15 mol.%, preferably from 7 mol.% to 11 mol.%, the molar fraction of (meth)acrylic acid monomeric units is from 2 mol.% to 8 mol.%, preferably from 3 mol.% to 7 mol.%, and the molar fraction of the crosslink is from 1 to 3 mol.%, molar fractions being the molar fractions in the crosslinked polymer.

According to another embodiment, the crosslinked polymer comprises copolymer chains having diethylene glycol methacrylate monomeric units, oligoethylene glycol methacrylate monomeric units comprising from 4 to 10 ethylene glycol moieties, and methacrylic acid monomeric units. The monomeric units are preferably: di(ethylene glycol) methyl ether methacrylate; oligo(ethylene glycol) methyl ether methacrylate having from 7 to 9 ethylene glycol moieties; and methacrylic acid. Oligo(ethylene glycol) methyl ether methacrylate monomeric units can also have from 8 to 9 ethylene glycol moieties.

The copolymer chains can be linked with a crosslink deriving from a crosslinking agent that may be selected from the group consisting of oligo(ethylene glycol) diacrylate comprising from 1 to 10 ethylene glycol units, 1,3-butanediol diacrylate, 1,4-butanediol diacrylate, 1,6-hexanediol diacrylate, pentaerythritol diacrylate monostearate, glycerol 1,3-diglycerolate diacrylate, neopentyl glycol diacrylate, poly(propylene glycol) diacrylate, 1,6-hexanediol ethoxylate diacrylate, trimethylolpropane benzoate diacrylate, ethylene glycol dimethacrylate, 1,3-butanediol dimethacrylate, 1,4-butanediol dimethacrylate, 1,6-hexanediol dimethacrylate, glycerol dimethacrylate, N,N-divinylbenzene, N,N'-methylenebisacrylamide, N,N-(1,2-dihydroxyethylene)bisacrylamide, poly(ethylene glycol) diacrylamide, allyl disulfide, bis(2-methacryloyl)oxyethyl disulfide and N,N-bis(acryloyl)cystamine.

The crosslinker has di(meth)acrylate end groups and a moiety selected in the group consisting of -(CH₂-CH₂-O)ₙ-CH₂-CH₂- where n is from 0 to 6, -NH-CH₂-NH- and mixtures thereof. The number n is preferably from 3 to 6.

The crosslinking agent is for example N,N'-methylenebisacrylamide, ethylene glycol)dimethacrylate, or oligo(ethylene glycol) diacrylate.

The crosslinking agent represents for example from 1 to 5 mol.% of the total number of moles of the three monomeric units.

A particular crosslinked polymer comprises diethylene glycol methacrylate monomeric units, oligoethylene glycol methacrylate monomeric units comprising from 8 to 9 ethylene glycol moieties, and a linker comprising di(meth)acrylate end groups and a moiety selected in the group consisting of -CH₂-CH₂-, -(CH₂-CH₂-O)ₙ-CH₂-CH₂-where n is from 4 to 5, and -NH-CH₂-NH-.

Inner structure of the microgels can depend on the crosslinker used. For example, active substances having a molecular weight that is lower than 1000 g/mol can be encapsulated into copolymer having -NH-CH₂-NH- bridges, active substances having a molecular weight that is between 1,000 and 4,000 g/mol can be encapsulated into copolymer having -CH₂-CH₂- bridges, and active substances having a molecular weight being from 4,000 to 10,000 g/mol can be encapsulated into copolymer having - (CH₂-CH₂-O)ₙ-CH₂-CH₂-where n is from 4 to 5 bridges.

The volume phase transition temperature (VPTT) of the loaded microgels according to the invention can be from 30 to 40°C, for example from 33°C to 36°C, or from 39°C to 41°C. The volume phase transition temperature (VPTT) of the unloaded microgels can be from 37 to 38°C. The volume phase transition temperature corresponds to the temperature at which the microgel particle structure changes from a collapse-state to a swelling-state.

The mean size of the microgels may vary depending on whether they contain water or not. The mean size of the microgels of the invention in the dry state may range from 100 to 1,000 nm. The hydrodynamic radial distribution function of the microgels measured at an angle of 60° and at a temperature of 20°C, can be less than 1.1 (monodispersed microgels).

The microgels of the invention can advantageously entrap cosmetic active substances that are soluble in water at 25°C (hydrophilic), or not (hydrophobic). Active substances can be soluble in an alcohol such as ethanol. Substances that are soluble in ethanol, (and eventually soluble in water) are particularly advantageous. The active substance can be solid or liquid at 25.0°C, meaning that it can have a melting temperature higher or lower than 25.0°C. The melting point can be determined by any suitable method known by one skilled in the art.

The microgels of the invention can encapsulate high amounts of different molecules: hydrophobic molecules, hydrophilic molecules, and macromolecules. The encapsulated amount and the released amount can be separately controlled depending on the active substance chemistry and desired biological release profile.

In a particular embodiment, active substances are selected from the group consisting of molecules having at least one -OH group that can have short-distance hydrophobic interactions, and molecules engaging hydrogen-bonding interactions with ether oxygen atoms of crosslinked copolymer in the microgels. Such interactions can be observed by Diffusion Ordered Spectroscopy NMR (DOSY-NMR) and Nuclear Overhauser Enhancement Spectroscopy (NOESY-NMR) measurements.

According to one embodiment, cosmetic active organic substances are hydrophobic molecules.

It is very surprising that encapsulation efficiencies in microgel formulations do not depend on the feeding substance solution concentration, in many cases.

The active substances may have a very high molecular weight that can be up to 50,000 g/mol, and can be for example from 50 to 30,000 g/mol, for example from 100 to 25,000 g/mol, from 50 to 6,000 g/mol, or from 50 to 2,000 g/mol.

Active substance molecules having at least one C=C double bond and at least one -OH group can create both hydrophobic and hydrophilic interactions with the crosslinked polymer. Such active molecules can further comprise at least one -COO-group. Active substance molecules can be aromatic compounds or not. They can also be selected from macromolecules such as polysaccharides.

The active substance may be preferably selected from the group consisting of UV filters, perfumes and anti-ageing active agents. The cosmetic active organic substance is selected from the group consisting of octyl salicylate, hyaluronic acid, diethylamino hydroxybenzoyl hexyl benzoate, benzophenone-4, citronellol and salicylic acid.

For example, the cosmetic active substance is selected in the group consisting of :
- 2-ethylhexyl salicylate (INCI octyl salicylate; sold for example under the tradename Escalol® 587; CAS Number 118-60-5), clear to pale yellow liquid, soluble in ethanol, slighty soluble in water,
- hyaluronic acid or sodium hyaluronate (having for example an average molecular weight Mw being from 1,000 to 50,000 g/mol, in particular 22,000 g/mol) liquid at 25°C, soluble et 15% in ethanol, soluble in water,
- hexyl 2-[4-(diethylamino)-2-hydroxybenzoyl]benzoate (INCI diethylamino hydroxybenzoyl hexyl benzoate; sold for example with tradename Uvinul® A; CAS number 302776-68-7) melting point being 54°C, soluble in ethanol, insoluble in water,
- 4-Hydroxy-2-methoxy-5-(oxo-phenylmethyl)benzenesulfonic acid (INCI benzophenone-4; CAS number 4065-45-6) powder at 25°C, soluble at 5% in ethanol, soluble at 5% in water,
- 3,7-dimethyloct-6-en-1-ol (INCI Citronellol, CAS Number 68916-43-8), liquid at 25°C, solubility in water being 200 mg/L at 25°C, very soluble in ethanol,
- 2-Hydroxybenzoic acid (INCI Salicylic acid, CAS Number 69-27-7), solid at 25°C, water solubility 2240 mg/L at 25 °C), soluble in ethanol.

The weight ratio of active substance to crosslinked polymer is from 500 microgram/mg to 10 mg/mg, for example from 500 microgram/mg to 8 mg/mg, still preferably from 500 microgram/mg to 6 mg/mg.

The weight ratio of active substance to crosslinked polymer is preferably lower than 10 mg/mg and higher than a lower limit selected in the group consisting of 550 microgram/mg, 600 microgram/mg, 650 microgram/mg, 700 microgram/mg, 750 microgram/mg, 800 microgram/mg, 850 microgram/mg, 900 microgram/mg and 1 mg/mg. According to one embodiment, the weight ratio of active substance to crosslinked polymer is higher than 550 microgram/mg.

The microgels of the invention can be prepared according to the steps of:
- preparing a dispersion of unloaded microgel particles in water,
- preparing a solution of the active substance,
- mixing the dispersion and the solution causing encapsulation of the active substance in the microgel particles, and
- recovering active substance loaded microgel particles.

Unloaded microgel particles in the sense of the present description contain no cosmetic active organic substances; they are essentially made of the crosslinked polymer and water.

Unloaded microgel particles are prepared for example by a precipitation polymerization method comprising a step of contacting in an aqueous phase, in the presence of a crosslinking agent, the three monomers described above, at a temperature of between 40°C and 90°C, preferably of the order of 70°C. The process of the invention does not require the presence of a surfactant such as SDS (dodecyl sulfate sodium), and polymerization may be initiated by addition of a water-soluble radical initiator, for example potassium persulfate (KPS).

Inner structure of the microgels can depend on the crosslinker used. According to several embodiments, three different microstructures were obtained: homogeneously cross-linked microgels using oligo(ethylene glycol) diacrylate (OEGDA), highly cross-linked shell and slightly cross-linked core microgels using N,N'-methylenebisacrylamide (MBA), and slightly cross-linked shell and highly cross-linked core microgels using (ethylene glycol)dimethacrylate (EGDMA).

The crosslinker can also influence the swelling ability of the microgel particles, the encapsulated amount and the release speed. The inner morphology of the microgels can be observed by 1H-nuclear magnetic resonance (1H NMR) and small-angle neutron scattering (SANS) techniques.

Active substance molecules can be encapsulated into microgels that are in the form of an aqueous dispersion, or into microgels that have been prepared in the form of a film according to the description above, in a prior step.

Mixing step of active substance solution and unloaded microgel dispersion preferably comprises a step of heating at a temperature that is higher than the volume phase transition temperature of the unloaded microgel particles, and a step of cooling the obtained dispersion of loaded microgels at ambient temperature (25°C).

The process of the invention advantageously enables a high entrapment efficiency EE% of the active substance, meaning that a very high proportion of the initial amount of active substance that is mixed with unloaded microgel particles (in the form of a aqueous dispersion of microgel particles, or in the form of a film of assembled microgel particles) is successfully entrapped within the microgel particles. According to the process of the invention, EE% is higher than a upper limit selected in the group of 50%, 60%, 70%, 80%, 90%, 95% when the amount of the active substance in the feeding substance is from 500 microgram/(1 mg unloaded microgels) to 10 mg/(1 mg unloaded microgels).

The feeding solution of the cosmetic active organic substance can be obtained by dissolution of a determined amount of the active substance in an appropriate solvent such as water or a solvent that is miscible with water, such as alcohols. Alcohols can be ethanol, propylene glycol, butylene glycol. Other solvents such as isododecane, isohexadecane, or decamethylcyclopentasiloxane can also be used.

A polar solvent that is soluble or miscible with water may be particularly advantageous-to enhance the loading amount of the active substance into the microgels.

Complete dissolution of a determined amount of the active substance in the solvent can be performed at a temperature being from ambient temperature to a temperature that is above the volume phase transition temperature of the unloaded microgel particles.

In a particular embodiment, a dispersion of unloaded microgel particles in water (0.1 to 10 mg particles/mL water) is heated at a process temperature. A solution of the active substance in a solvent (0.5-125 mM or 0.5-2.5 mM) is heated at this process temperature as well, and then mixed with the unloaded microgel particle aqueous dispersion under stirring while maintaining the same temperature. The process temperature can be a temperature that is higher or lower that the VPTT of unloaded microgel particles, the particles being respectively in collapse or swollen state. For example, the process temperature is at least 10°C higher, preferably at least 15°C higher than the VPTT, or at least 10°C lower, preferably at least 15°C lower than the VPTT. Removal of the solvent and removal of active substance molecules that have not been trapped into the microgel particles can be performed subsequently, in order to obtain microgels according to the invention. Removal of active molecules that have not been entrapped can be performed by filtering and/or by centrifugation.

The "Entrapment efficiency (EE%)" that is defined as the ratio of the amount of the cosmetic active organic substance in the loaded microgels and the amount of the cosmetic active organic substance in the feeding solution, can be modulated according to the loading process conditions such as pH, temperature and solvents.

The Entrapment efficiency (EE%) is advantageously from a lower limit selected in the group consisting of 50%, 55%, 60%, 65%, 70%, 75%, 80%, 85%, 90%, 95%, to a upper limit of 100%.

In the prior art, EE% is usually above 50% for a feeding substance amount that is lower than an upper limit of 200 microgram/mg of unloaded microgel particles. Above this upper limit, EE% usually drops to 15% or even lower. The microgels of the invention can advantageously entrap a high percentage of the feeding substance amount, even for high feeding substance amount being from 500 microgram/(mg unloaded microgel particles) to 5 mg/(mg unloaded microgel particles), or from 1 mg/(mg unloaded microgel particles) to 10 mg/(mg unloaded microgel particles). There is no saturation effect of the loading of the microgel particles with active substance as observed in the prior art with other microgels. In the present description, the feeding substance amount unit is "mg/(mg unloaded microgel particles)" and can also be written "mg/mg".

For example, EE% is higher than a upper limit selected in the group of 50%, 60%, 70%, 80%, 90%, 95% when the feeding substance amount is from 500 microgram/mg to 10 mg/mg.

In one particular embodiment, EE% is higher than 80% for a feeding 2-ethylhexyl salicylate amount being from 10 microgram/mg to 10 mg/mg.

In the case of hyaluronic acid (having for example a molecular weight being from 1,000 to 50,000 g/mol) as an active substance, EE% can be higher than 50% using a feeding substance amount being from 300 microgram/mg to 1 mg/mg.

The active substance can also be hexyl 2-[4-(diethylamino)-2-hydroxybenzoyl]benzoate or salicylic acid. In that case, EE% can be higher than 80% for a feeding substance amount being from 1 mg/mg to 10 mg/mg.

Active substance molecules can be encapsulated into microgels that are in the form of a film having a thickness being from 100 to 400 microns. The present application also discloses films comprising loaded microgel particles as disclosed above.

Oligo(ethylene glycol) methacrylate unloaded microgels that are used to prepare loaded microgels of the present invention have the advantage of self-assembling in one or more layers of particles by simple evaporation of water that is contained in an aqueous dispersion of particles at ambient temperature.

The invention also relates to a process for the preparation of microgels as described above, said process comprising a step of preparing a feeding solution of the cosmetic active organic substance in a solvent, a step of preparing an aqueous dispersion of unloaded microgel particles, a step of mixing the solution and the aqueous dispersion under stirring so as to entrap the substance into the unloaded microgel particles, and a step of recovering the microgels.

The invention further related to a process for the preparation of cosmetic active organic substance loaded microgels in the form of a film, said process comprising the steps of:
- preparing a feeding solution of the cosmetic active organic substance in a solvent,
- preparing a film of unloaded microgel particles,
- immersing the film in the feeding solution so as to cause swelling of the film and diffusion of the active substance into the film, and
- recovering the microgels that can be in the form of an active substance loaded microgel film.

The films of unloaded microgel particles (also called bare microgel films) can be formed according to a step of placing a water dispersion of loaded microgel particles that can be prepared according to the process as described above into a mold (such dispersions can have a pH value being from 4 to 7.5), and a step of drying the water dispersion. Drying can be performed by placing the mold at a temperature higher than 30°C or being ambient temperature.

The step of immersing the film can be performed at 25°C for at least 12 hours or 24 hours.

Oligo(ethylene glycol) methacrylate unloaded microgels that are used to prepare loaded microgels of the present invention are also capable of forming a transparent film and capable of forming a cohesive and elastic film. It is not necessary in the context of the invention to encapsulate or support the microgels in order to form a film; consequently, interaction between the microgels and skin on which they are formed after water evaporation of an aqueous dispersion of the microgel particles is optimal.

Microgel films of the present invention are very useful for skincare applications because they can interact with skin as smart delivery systems while fulfilling advanced properties such as surface protection, mechanical and optical properties. In addition, skin irritation can be avoided with the films that have higher water vapor permeation through skin properties.

The present invention also concerns the microgels as described above for the delivery of cosmetic active organic substance, in particular the dermal delivery of a cosmetic active organic substance. Delivery of cosmetic active hydrophobic organic substances is particularly advantageous in the context of the invention.

The delivery being an immediate release, a sustained release, a controlled release, an extended release or a targeted release of the cosmetic active organic substance into a release medium, at the skin surface, into the epidermis or into the dermis. The delivery can be a pH triggered delivery, a temperature triggered delivery or a solvent triggered delivery. Delivery kinetics may vary depending on pH, temperature, composition of the releasing medium (ethanol, polar solvent, surfactant) or crosslinker used to prepare the bare microgels.

The inventors have found that various releasing profiles can be obtained. A continuous release of the active substance out of the microgels can be observed for at least 6 hours, at least 12 hours, at least 24 hours or even at least 48 hours. At the end of this period, the release can stop and a maximum total release percentage of the active substance can be lower than 100% and higher than a value selected in the group consisting of 10%, 15%, 20%, 25%, 30%, 35%, 40%, 45%, 50%, 55%, 60%, 65%, 70%, 75%, 80%, 90% and 95%.

In a particular embodiment, the cosmetic active organic substance that is initially comprised in the microgels, goes out of the microgels into a release medium at the end of a period starting from the contact of loaded microgels. More in detail, the cosmetic active organic substance can go out of the microgels into a release medium in an amount corresponding to a release percentage that is lower than 100% and higher than a value selected in the group consisting of 10%, 15%, 20%, 25%, 30%, 35%, 40%, 45%, 50%, 55%, 60%, 65%, 70%, 75%, 80%, 90% and 95%. The period can be selected form the group consisting of at least 6 hours, at least 12 hours, at least 24 hours and at least 48 hours, after that the microgels are put into contact with the release medium. The release medium can have different features: a pH being from 4.5 to 7.4, a ionic force being from 1 mM to 20 mM, and/or a temperature being 25°C or 37°C. The release medium can have a pH being from 5.0 to 6.0.

Moreover, the crosslinking distribution can be tuned by a suitable choice of the crosslinker. Microgels with different internal structures can be obtained, from homogeneously crosslinked microgels, microgels with densely-crosslinked core and loosely-crosslinked shell microstructure, microgels exhibiting slightly crosslinked network with densely-crosslinked thin shell.

Delivery profile of the active substance from the loaded microgels can be tuned by selection of microgel structure and active substance encapsulation amount. The active substance release can be influenced by the microgels microstructure as well as by the amount of active molecule encapsulated, the release being faster in the case of homogeneously cross-linked microgel particles loaded with high amounts of active molecules.

*In vitro* release profiles have showed that cosmetic active molecules release can be controlled by the pH (being for example pH 4.5, pH 6 or pH 7.4), by temperature of the medium (being 25°C or 37°C) as well as by the medium hydrophobicity (presence of ethanol or presence of a surfactant in the dialysate medium).

In addition, at those conditions where microgel particles are swollen, the cosmetic active molecules release may be controlled by Fickian diffusion and Case-II transport, being the diffusional process dominant. These results indicate that multi-responsive oligo(ethylene glycol)-based microgels are cosmetic active delivery systems.

Another object of the present invention is a cosmetic composition comprising the microgels as described above, a perfume and a preservative.

The cosmetic composition can be in the form of a make-up product, a skin care product or a UV protecting product, and can further comprise any cosmetic excipient such as a compound selected in the group consisting of oils, surfactants, buffers, solvents, pigments and dyes, such a compound not being entrapped in the microgels. The delivery of microgels according to the invention is of particular interest for cosmetic composition having a ionic strength being from 1 mM to 20 mM, for example from 15 to 20 mM.

The present invention also deals with a non-therapeutic cosmetic treatment method comprising a step of applying on skin, nails, lips or hair of a person, microgels as described above or a cosmetic composition as described above. All the features applying to the microgels and all the features applying to the cosmetic composition that have been described before also apply to the non-therapeutic cosmetic treatment method.

A non-therapeutic skin care cosmetic method and a cosmetic make-up method comprising a step of applying on skin, nails, lips or hair of a person, a product selected form the group consisting of i) a cosmetic composition comprising microgels in the form of an aqueous dispersion, ii) a cosmetic composition comprising microgels in the form of film, iii) microgels in the form of a dispersion in a solvent, or iv) microgels in the form of a film, and mixtures thereof, are also part of the present disclosure.

### BRIEF DESCRIPTION OF THE FIGURES

FIG.1 presents Uvinul-A encapsulated amount as a function of Uvinul-A feeding solution concentration with OEGDA microgel.
FIG.2 presents Escalol encapsulated amount as a function of Escalol feeding solution concentration with OEGDA microgel.
FIG.3 and FIG.4 displays encapsulated amounts of Uvinul-A and Salicylic Acid as a function of feeding solution concentration for OEGDA microgel, EGDMA microgel and MBA microgel.
FIG.5 displays encapsulated amounts of Citronellol and Hyaluronic acid active molecules as a function of feeding solution concentration with OEGDA microgel.
FIG.6 displays encapsulated amounts of Benzophenone-4 as a function of feeding solution concentration for OEGDA microgel.
FIG. 7 displays encapsulated amount of Salicylic Acid as a function of feeding solution concentration for OEGDA microgel.
FIG.8 provides with encapsulated amount of Uvinul-A as a function of feeding solution concentration for OEGDA self-assembled microgel film.

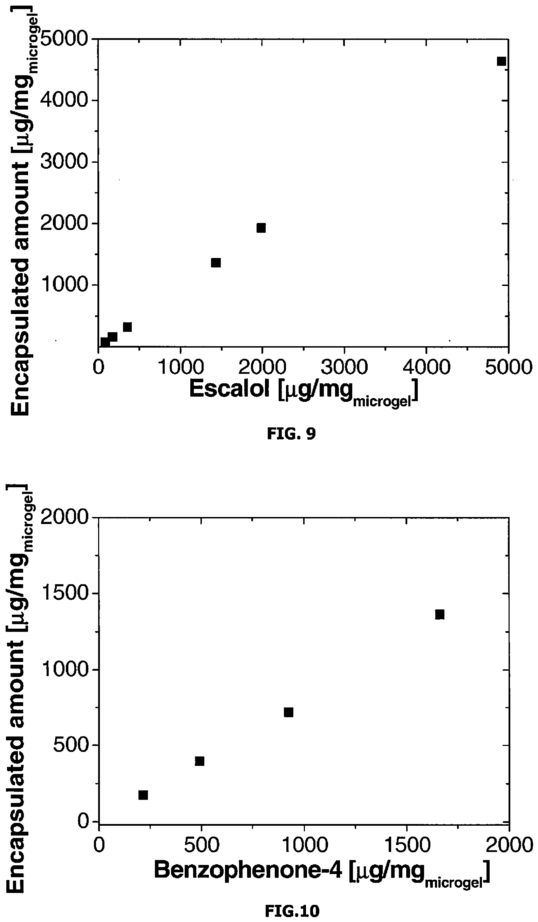

FIG. 9 displays encapsulated amount of Escalol as a function of feeding solution concentration for OEGDA self-assembled microgel film.
FIG.10 displays encapsulated amount of Benzophenone-4 as a function of feeding solution concentration for OEGDA self-assembled microgel film.
FIG.11 displays encapsulated amount of Salicylic Acid as a function of feeding solution concentration for OEGDA self-assembled microgel film.
FIG. 12 displays Salicylic Acid time release as a function of microgel microstructure depending on crosslinker type.
FIG.13 displays Uvinul-A time release as a function of cross-linker type.
FIG.14 is the time release curve of Benzophenone-4 as a function of temperature at pH 4.5.
FIG.15 is the release curve of Salicylic Acid as a function of temperature at pH 4.5.
FIG.16, FIG. 17, FIG. 18a and FIG.18b are time release profiles of Salicylic acid, Benzophenone-4, Escalol and Uvinul-A as a function of ethanol content in the release medium.
FIG. 19a, FIG. 19b, FIG. 19c and FIG. 19d respectively present Uvinul-A, Escalol, Benzophenone-4 and Salicylic acid release with time as a function of release medium pH.
FIG. 20a, FIG. 20b, FIG. 20c and FIG. 20d respectively present Uvinul-A, Escalol, Benzophenone-4 and Salicylic acid time release profiles as a function of microgel dispersion pH and release medium pH.
FIG. 21a, FIG. 21b, FIG. 21c and FIG. 21d respectively present Uvinul-A, Escalol, Salicylic acid and Benzophenon-4 acid time release profiles in different receiving solutions.

### EXAMPLES

### MATERIALS

Di(ethylene glycol) methyl ether methacrylate (MeO₂MA 95%, Aldrich), oligo(ethylene glycol) methyl ether methacrylate (OEGMA, monomethyl terminated with 8 EG repeat units, number average weight Mn = 475 g mol⁻¹, Aldrich), N,N'-methylenebisacrylamide (MBA, Aldrich), (ethylene glycol)dimethacrylate (EGDMA, Aldrich), methacrylic acid (MAA, Aldrich), oligo(ethylene glycol) diacrylate (OEGDA, number average weight Mn = 250 g mol⁻¹, Aldrich), potassium persulfate (KPS 99%, ABCR), and ethanol (VWR Chemicals) were used as received. Hydrochloric acid (HCI, 36 w/w, ABCR) and potassium hydroxide (KOH, Aldrich) were used to control the pH of dispersions. Citric acid (Sigma-Aldrich) and sodium phosphate dibasic (Na₂HPO4, Sigma-Aldrich) were used to prepare the buffers.

### EXAMPLE 1: Encapsulation of cosmetic active molecules into bare microgel dispersion

### Synthesis of bare microgel dispersion

83.90 mmol of MeO₂MA, 9.36 mmol of OEGMA, 1.92 mmol of a cross-linker and 930 g of "Milli-Q" grade water were placed into a 2 L jacketed glass reactor. The cross-linker can be OEGDA, MBA or EGDMA. The reactor content was stirred at 150 rpm and purged with nitrogen for 45 min to remove oxygen at room temperature. Then, 5 mmol of MAA dissolved in 30 mL of "Milli-Q" grade water were added to the jacketed glass reactor and the mixture was heated up to 70°C. After adding the initiator (0.89 mmol of KPS dissolved in 40 mL of degassed water), the polymerization reaction was allowed to continue under nitrogen atmosphere while stirring for 6 h. The reaction mixture was subsequently cooled to 25 °C maintaining the stirring, and the final dispersion was purified by centrifugation / redispersion cycles (10,000 rpm, 30 min) with "Milli-Q" grade water. A OEGDA-microgel dispersion, a MBA-microgel dispersion or a EGDMA-microgel dispersion is obtained.

### Encapsulation process

A cosmetic active substance was loaded into a OEGDA-microgel dispersion, a MBA-microgel dispersion or a EGDMA-microgel dispersion prepared as described above.

The active substance could be diethylamino hydroxybenzoyl hexyl benzoate (Uvinul® A also named Uvinul-A in the present description), octyl salicylate (Escalol® 587 also named Escalol in the present description), benzophenone-4, hyaluronic acid, citronellol or salicylic acid.

### a- Encapsulation process for Uvinul® A, octyl salicylate, salicylic acid, benzophenone-4 and citronellol

A OEGDA-microgel dispersion, a MBA-microgel dispersion or a EGDMA-microgel dispersion (1 mg/L) was heated to and incubated at 50°C (above the volume phase transition temperature VPTT) for 30 min. To this microgel dispersion different preheated concentrations of the active molecules in ethanol (0.5-2.5 mM) were added under magnetic stirring.

The mixture was stirred for 30 min at 50°C (above the VPTT) or at 20 °C (below the VPTT). After that, the mixed dispersion was stirred overnight at 50 °C to remove the organic solvent.

Dispersion was filtered to remove the unloaded active molecule precipitate and the filter was washed with ethanol twice, obtaining a solution containing unloaded Uvinul-A, unloaded salicylic acid or unloaded Benzophenone-4. Unloaded active molecules amounts were determined by UV-Vis.

For all other compounds, samples were centrifuged during 30 min at 10,000 rpm after overnight incubation to recover the aqueous supernatant.

In the case of octyl salicylate, the aqueous supernatant was evaporated using a rotavap and the free octyl salicylate molecules were dissolved in ethanol to determine non-encapsulated amount by UV-Vis.

In the case of citronellol, aqueous supernatant containing free citronellol molecules was analyzed by 1H NMR.

### b- Encapsulation process for hyaluronic acid:

Microgel particles were lyophilized and suspended in different solutions of hyaluronic acid in water at particle concentration of 1 mg/mL. Then, the microgel particles were allowed to rehydrate for 12 h at room temperature while shaking. Microgel particles were separated from the aqueous medium containing by centrifugation and the equilibrium active molecule concentration was determined by ATR/FTIR.

### Measurement of microgel VPTT

Loaded microgels were prepared according to the above protocole at 20°C.

VPTT of loaded OEGDA-microgel dispersions were different depending on the type of encapsulated cosmetic active molecule (see Table 1). VPTT values were lower for encapsulated Uvinul-A and Escalol.

**Table 1 - VPTT values for bare and loaded-microgel particles**

| **Sample** | **VPTT (°C)** |
|---|---|
| **Bare microgel** | 37.4 |
| **Uvinul-A-loaded microgel** | 35.0 |
| **Escalol-loaded microgel** | 35.1 |
| **Benzophenone-4-loaded microgel** | 39.9 |
| **Salicylic acid-loaded microgel** | 39.9 |

These results are very interesting from the point of view of cosmetic applications since, thanks to the thermal behavior of loaded-microgel particles as a function of pH, the release of different active molecules can be controlled by medium temperature and pH, confirming the results discussed above.

From the point of view of cosmetic applications one of the most interesting properties of oligo(ethylene glycol)-based microgels is their pH-sensitive thermal behavior.

### Encapsulation efficiency of Uvinul-A and Escalol into OEGDA-microgel dispersions

Uvinul-A-loaded OEGDA-microgels and Escalol-loaded microgels were prepared according to the above protocole at 20°C and at 50°C.

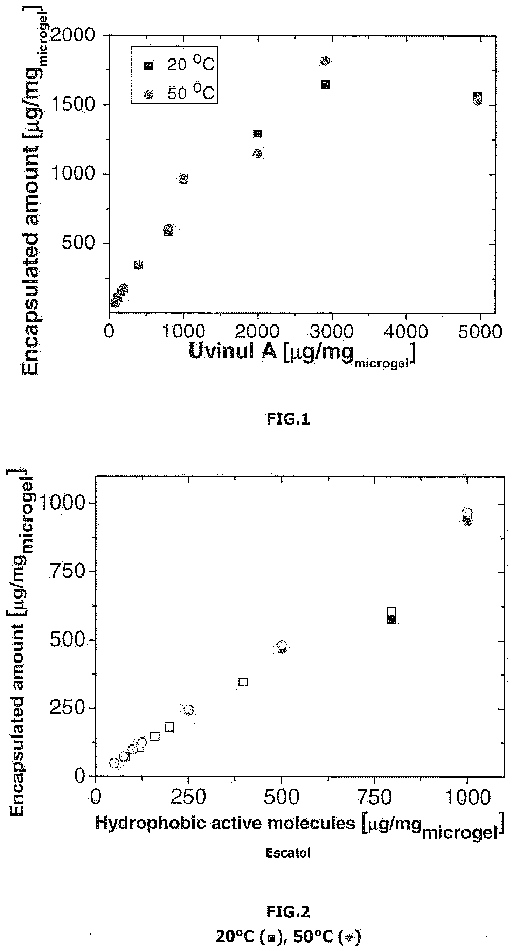

Encapsulated amount of Uvinul®A increases as the feeding substance amount increases until 3.000 µg/mg microgel, whatever the encapsulation temperature may be (above or under VPTT of the bare microgels). E.E. % is higher than 70% when the feeding substance amount is lower than 1,500 µg/mg microgel. E.E. % is higher than 50% when the feeding substance amount is between 1,500 and 3,000 µg/mg microgel (see Figure 1).

The encapsulated amount increases as the Escalol concentration increases, does not depend on the encapsulation temperature. E.E. values are above 90% in all cases (see Figure 2). Moreover, E.E. values above 70 % were obtained independently of the type of hydrophobic active molecule and encapsulation temperature used.

This behavior was different to that observed for different hydrophobic drugs by Qiao et al., J. Control. Release, 152, (2011), 57-66*.* They observed that loading temperature, being higher above the VPTT of the nanogels, influenced encapsulation efficiency.

The driving force to encapsulate Uvinul-A and Escalol molecules into P(MeO₂MA-OEGMA-MAA) microgel particles could be the hydrophobic interactions as well as the interactions by H-bonding between the -OH groups of both cosmetic active molecules and the ether oxygen of the ethylene glycol units of microgel.

### Comparison of OEGDA-microgel, MBA-microgel and EGDMA-microgel dispersions with regard to encapsulation of Uvinul-A and Salicylic Acid

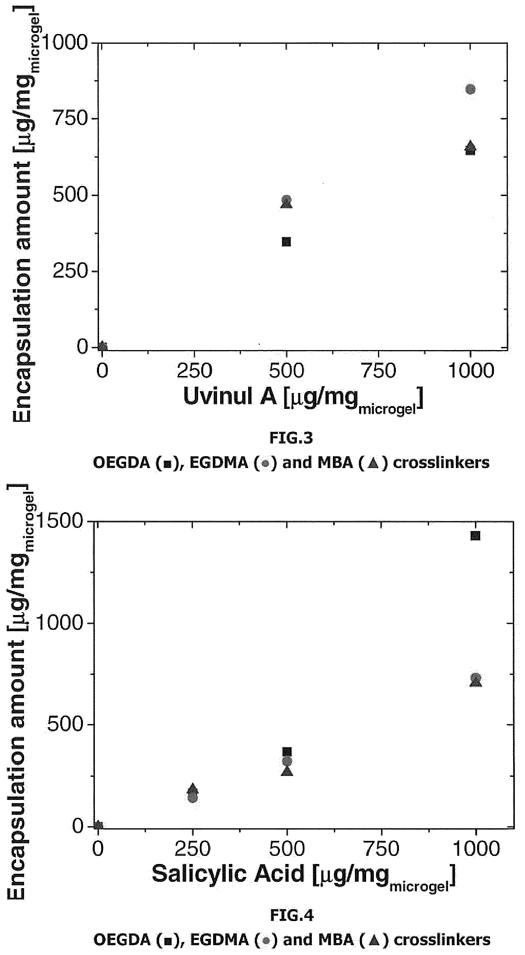

Uvinul-A and Salicylic Acid were encapsulated into a OEGDA-microgel dispersion, a MBA-microgel dispersion or a EGDMA-microgel dispersion at 20°C (see Figure 3 and Figure 4).

In the case of hydrophobic Uvinul-A, the inner morphology of microgel particles has an effect on encapsulation efficiency being the E.E. the lowest one in the case of hydrophilic MBA and highly cross-linked shell that could hinder the entering of Uvinul-A molecules.

In the case of Salicylic Acid encapsulation, taking account the experimental error of these measurements (∼10%), it can be concluded that there is no effect of microgel microstructure on active molecules encapsulation.

### Encapsulation efficiency of Citronellol and Hyaluronic acid into OEGDA-microgel dispersions

Encapsulation of Citronellol and Hyaluronic acid into homogeneously cross-linked (using OEGDA cross-linker) microgel particles was studied. Citronellol is a natural acyclic monoterpenoid (without aromatic ring and therefore having no hydrophobic interactions with the crosslinked polymer) and Hyaluronic acid is a hydrophilic polysaccharide (macromolecule) which structure contains repeating units of D-glucuronic acid and N-acetyl-D-glucosamine.

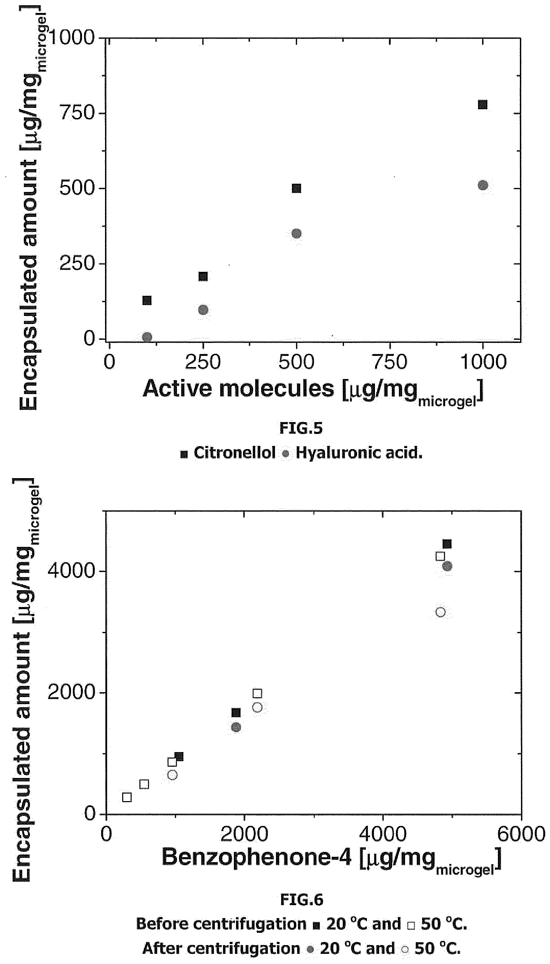

The encapsulated amounts of both molecules increased linearly with their concentration (see Figure 5).

In the case of Citronellol it seems that hydrogen bonds were enough to obtain a good encapsulation of it (E.E. > 70 %).

In the case of Hyaluronic acid the E.E. values were lower than those observed in the case of small cosmetic active molecules (Uvinul-A, Salicylic acid, and Citronellol). At pH 6 (encapsulation pH), hyaluronic acid molecules were negatively charged (pKa = 3) as were homogeneously cross-linked microgel particles; therefore, this could lead in an electrostatic repulsion between hyaluronic acid molecules and microgel particles causing a more difficult encapsulation. This together with the larger size of this macromolecule could be the reasons of 50% E.E. values at 600 microgram/mg.

However, the total amount of macromolecule loaded was much higher to that observed in the case of using other polymeric delivery systems to encapsulate macromolecules. For example, Cun et al. (Eur. J. Pharm. Biopharm., 2011, 77, 26*)* studied the encapsulation of siRNA molecules into poly(DL-lactide-co-glycolide acid) (PLGA) nanoparticles obtaining E.E. values of 70 % and siRNA encapsulated amounts of around 2 microgram/mg.

### Encapsulation efficiency of Benzophenone-4 and Salicylic acid into OEGDA-microgel dispersions

### - Benzophenone

Ethanol is used in the encapsulation process as described before, at 20°C and at 50°C. Loaded microgel dispersion was centrifuged and the supernatant was analyzed by UV-Vis.

As benzophenone-4 is water soluble, part of the non-encapsulated Benzophenone-4 could be soluble in water phase. In order to quantify non-encapsulated benzophenone and water solubilized active molecule amount, encapsulation amounts were calculated before the centrifugation step and after the centrifugation step. The encapsulation process was performed.

As can be observed, part of the non-encapsulated Benzophenone-4 is in the water phase: therefore, the encapsulated amount (see Figure 6) and E.E. values are lower after centrifugation than before centrifugation. It is necessary to perform the centrifugation step in order to quantify the total non-encapsulated Benzophenone-4 amount.

The encapsulated amounts increased linearly with the active molecule concentration and E.E. values were higher than 80%, in all the cases as long as the feeding substance amount is lower than 1 mg/(mg unloaded microgels) (see Figure 6).

### - Salicylic acid

The same study has been performed with salicylic acid as the cosmetic active substance.

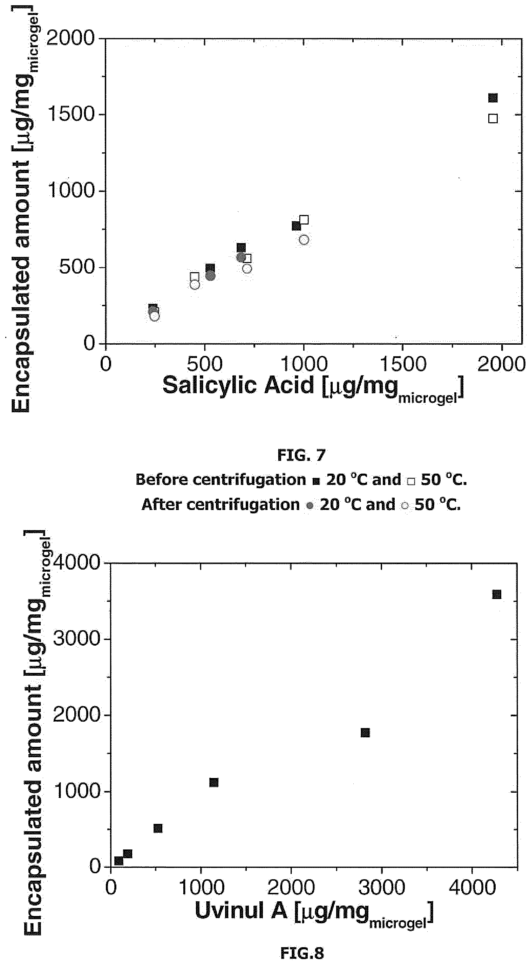

In Figure 7, increasing the concentration of Salicylic Acid, the E.E. value and the encapsulated amount of it increase. In addition, as in the case of Benzophenone-4, centrifugation step is necessary in order to quantify the total non-encapsulated Salicylic Acid amount.

### EXAMPLE 2: Encapsulation of cosmetic active molecules into bare microgel films

### Preparation of the bare microgel films

Films composed of multilayer of self-assembled microgel were formed as follows: 30 mL of an aqueous OEGDA-microgel dispersion as prepared above (presence of water soluble polymers or not) and having different solid content (such as 1.4 wt%) was introduced into inert plastic mold and dried for 48 h at 35 °C (±3 °C) at atmospheric pressure.

Ability of unloaded microgel particles to self-assemble and to form cohesive films has been demonstrated at different pHs and in presence of different types of salts (citric/sodium phosphate dibasic at pH 4.5, at pH 6 and at pH 7; potassium carbonate at pH 9). Cohesive films can be formed from aqueous dispersion of bare microgels aqueous dispersions having different pH and ionic strength.

Film stability in hydrated state was studied through the immersion of self-assembled microgel films into aqueous solution at room temperature for 24 h. Swelling of the film is observed instead of redispersion of microgels. Furthermore, self-assembled microgel films present a reversible swelling-deswelling process without losing its form. The reason could be the existence of elastic forces between the oligo(ethylene glycol) polymer chains preserving the cohesion between microgel particles.

Different medium conditions were prepared in order to evaluate swelling behavior of films. Hydrophobicity (water/ethanol and water/isopropanol), temperature, pH, and purification of water soluble polymer obtained as a by-product of the microgel particles preparation were medium criteria. Above the VPTT, swelling ability of the film in hydrophobic medium is higher than in hydrophilic one. However, swelling ratio is lower in the case of the films formed in presence of water soluble polymer by product that create osmotic pressure.

### Encapsulation of Uvinul®-A, Escalol, salicylic acid and benzophenone-4 molecules into bare microgel films

Uvinul® A, Escalol, salicylic acid and benzophenone-4 molecules were separately encapsulated into unloaded films composed of multilayer of self-assembled unloaded microgel as previously prepared.

A solution containing one of the four active substances in a water/ethanol (75/25) mixture was prepared, and the films were immersed in this feeding solution allowing the film to rehydrate for a 24 h period of time.

Salicylic acid loading into self-assembled microgel films was studied in media having different pH. Self-assembled microgel films were rehydrated in buffered media containing different concentrations of active molecules during 24 hours.

Encapsulated amount of the active substance in the films was evaluated by UV-Vis characterization or ATR-FTIR characterization recorded on a Spectrum One (PerkinElmer) spectrometer.

Transmittance data of loaded-films were collected using Shimadzu UV-2101 spectrometer from 300 to 500 nm. Loaded-films were enough sticky to hold themselves to sample holder and therefore, air was used as reference. Four scans were made for each measurement and all spectra were recorded at 25 °C and atmospheric pressure.

Above VPTT, short-distance hydrophobic interactions between self-assembled microgel film and Uvinul® A (or Escalol) became more intense. Opposite is observed with Salicylic acid or Benzophenone-4.

Encapsulation efficiencies (E.E.) were above 70% in all cases. Entrapment efficiency is about 80% for every Benzophenone-4 concentration.

Independently of the type of active molecule used, encapsulated amount increases as the concentration of active molecule increases.

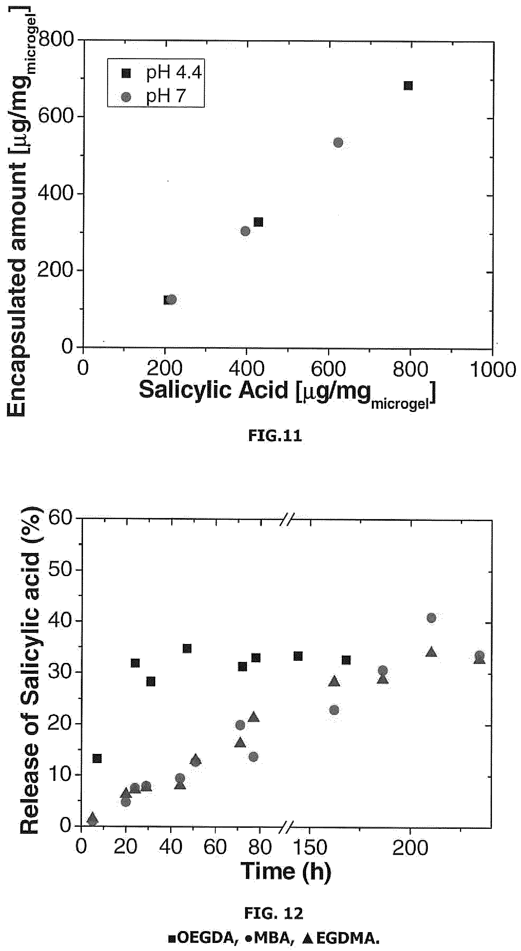

As can be seen in Figure 11, encapsulated amount and E.E increase as the concentration of Salicylic Acid increases at both pHs. The same behavior was observed in the case of using microgel particles. In addition, it seems that there is no influence of the pH of the medium on the active molecule loading.

### EXAMPLE 3: in vitro release of cosmetic active molecules from loaded microgels

The study of active molecules release kinetics was carried out by a dialysis method in order to verify that no microgel diffusion occur in dialysate medium (that may also be called "release medium"), and in order to observe the cosmetic active molecule release profile against time.

For that, active molecules-loaded microgel particle dispersions (1 mg/mg microgel) were dialyzed against a dialysate medium. Diffusion of the cosmetic active molecule from the loaded-microgel dispersion medium to the dialysate medium is observed, until an equilibrium concentration is reached between both media.

In vitro cosmetic active molecule percentage release results are represented in the form of curves as a function of time in Figures 12 through to 21.

If not mentioned otherwise in the examples, loaded microgel dispersions are loaded OEGDA-microgel dispersion (meaning that the crosslinker that is used to prepare the bare microgel particles is OEGDA).

### Methods

### i) In vitro release by a dialysis method

Loaded-microgel particles comprising an active molecule in an amount of 1 mg /(mg unloaded microgel) were placed inside a dialysis tube and dialyzed in different buffered dialysate media (having different pH, having different temperature, containing a solvent and/or containing a surfactant). The buffered dialysate medium can contain ethanol, citric acid, sodium phosphate dibasic or/and polyoxyethylene monooleate sorbitan (also named Polysorbate 80, Tween® 80 or INCI Sorbitan oleate). Active substance concentration in the dialysate medium was determined by UV-Vis.

### ii) In vitro release by real-time ATR/FTIR spectroscopy

In vitro release can alternatively be followed by real-time ATR/FTIR spectroscopy. In this case, no dialysis membrane is placed in the dispersion.

Loaded-microgel particles comprising 1 mg active molecule/(mg unloaded microgels) were placed in a buffered medium at pH 6 and 25 °C. In situ ATR/FTIR monitoring was performed using a ReactIR 15 with a diamond attenuated total reflection DiComp probe and equipped with a liquid nitrogen cooled MCT detector. Spectra were collected directly in the release medium at different incubation times.

### Effect of microgel microstructure on cosmetic active molecule time release profile

### a) Salicylic acid

Three different microgel dispersions loaded with an active molecule were used: a salicylic acid loaded OEGDA-microgel dispersion (homogeneous crosslinked particles), a salicylic acid loaded MBA-microgel dispersion (loosely cross-linked core and highly cross-linked shell) and a salicylic acid loaded EGDMA-microgel dispersion (highly cross-linked core and loosely cross-linked shell). AS explained before, a "OEGDA-microgel dispersion" means that bare microgel particles have been prepared with a OEGDA crosslinker.

The homogeneous distribution of the cross-linker (OEGDA) inside microgel particles promoted a faster release of Salicylic acid between T0 and T40H. After T160H the same amount of active molecule was released in all three cases (see Figure 12). Therefore, it seems that the microstructure of the microgels has an effect of Salicylic Acid release kinetics but not in the amount released.

### b) Uvinul-A

Three different microgel dispersions loaded at a 1 mg active molecule/(mg microgel) concentration were used: a Uvinul-A loaded OEGDA-microgel dispersion, a Uvinul-A loaded MBA-microgel dispersion and a Uvinul-A loaded EGDMA-microgel dispersion.

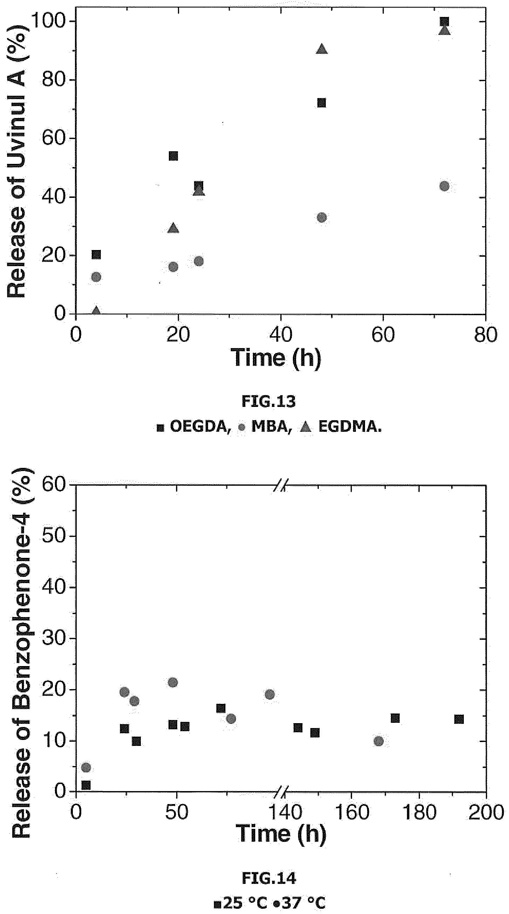

In vitro Uvinul-A release was followed by real-time ATR-FTIR spectroscopy. The spectra were collected directly in the release medium (25 °C and pH 6) at different incubation times (see Figure 13).

### Effect of temperature on time release profile

The effect of temperature on the release kinetics of active molecules was investigated with the dialysate method maintaining pH of the dialysate medium constant at 4.5. The in vitro release was studied at two different temperatures: 25°C (below the VPTT) and 37°C (above the VPTT).

In the case of Benzophenone-4, it seems that the temperature has no effect on the release kinetics.

On the other hand, in the case of Salicylic Acid, the release is higher at 25 °C (being microgel hydrophilic) than that at 37 °C (being microgel hydrophobic).

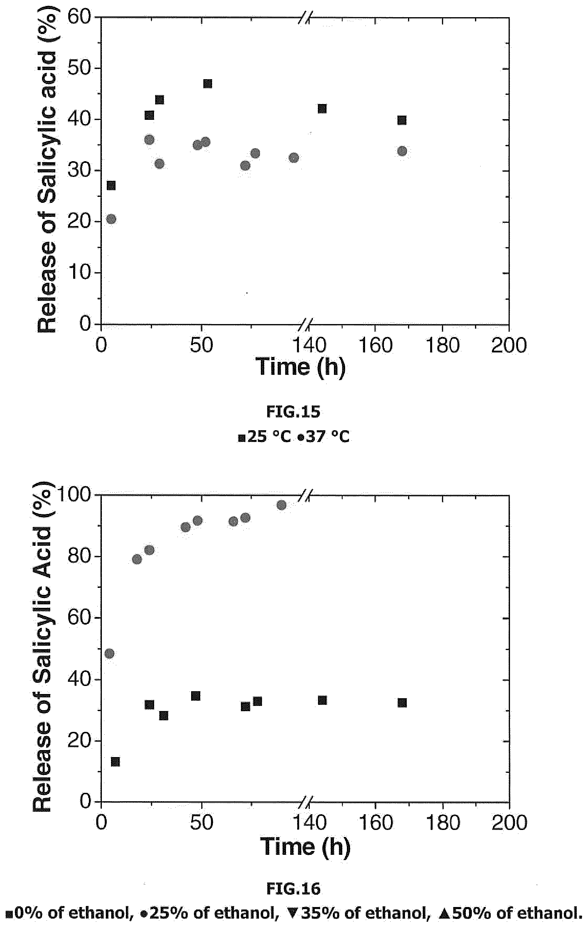

The results are presented in Figure 14 and in Figure 15.

### Effect of hydrophobicity of the release medium on time release profile

Four different active molecules loaded-microgel dispersion were tested: salicylic acid, benzophenone-4, Escalol and Uvinul-A.

Hydrophobicity of the dialysate medium was varied according to the dialysis method. By adding different amounts of ethanol (0%, 25%, 35% or 50% ethanol) in the dialysate medium maintaining the pH and the temperature constant (pH 6 and 25°C).

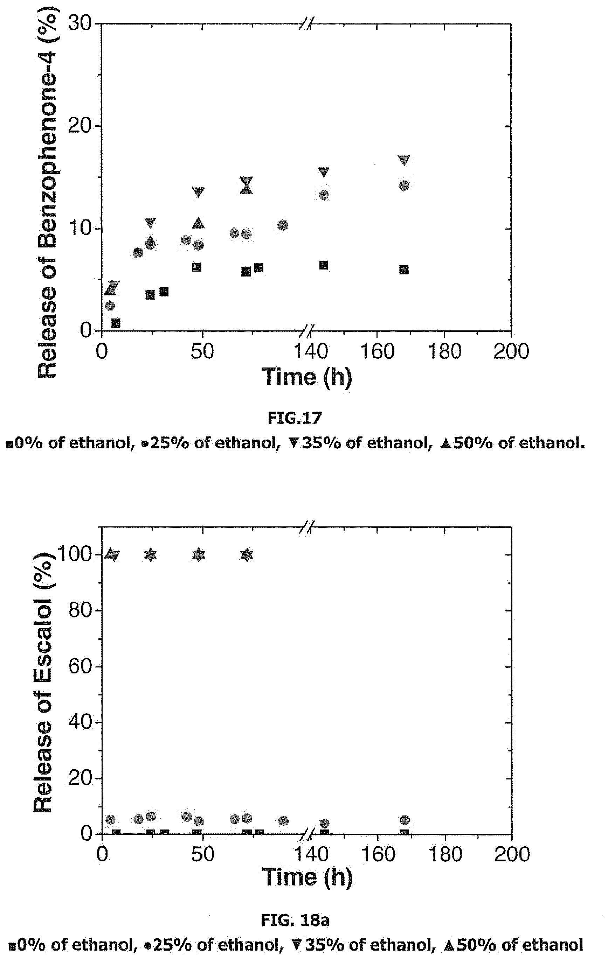

The complete release of Salicylic acid is obtained increasing the ethanol percentage from 0 to 25% (see Figure 16). By contrast, in the case of Benzophenone-4, the effect of the ethanol percentage on release kinetics is lower and the complete release of it is not achieved, in any case. In addition, the Benzophenone-4 release is higher at 35% of ethanol than that at 50% (see Figure 17).

The active molecules release of hydrophobic molecules Uvinul-A and Escalol increases with an increasing ethanol percentage, in all the cases. These results are expected since increasing ethanol percentage causes hydrophobicity of the dialysate medium to increase, thereby enhancing the release of hydrophobic active molecules.

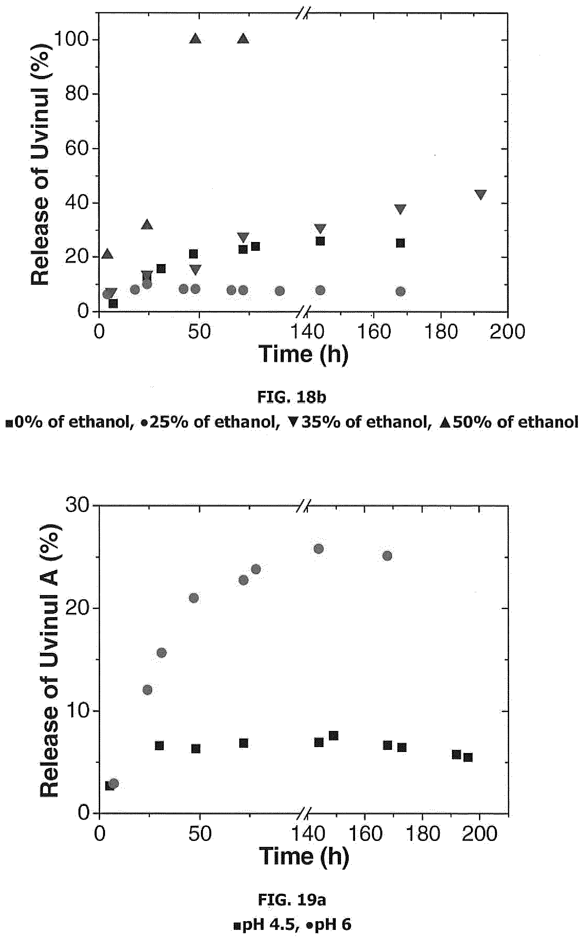

At an ethanol percentage of 35% and a 168 h incubation time, active molecule release increases from 20% to 40% for Uvinul-A, and from 10% to 100% for Escalol. In the case of Escalol the release is not sustained since 100% is released in the first 6 hours of incubation (see Figure 18a and Figure 18b).

### Effect of release medium pH on active substance time release profile

a) With the aim of studying pH effect on *in vitro* active molecule release, loaded microgel particles (1mg/mg microgel) were placed into different buffered dialysate media (1mM at pH 4.5 or at pH 6) at 25°C. Several active molecules were studied. pH of the loaded-microgel dispersion was not varied.

In Figure 19a, a plateau is reached at all pHs. It seems that the equilibrium between Uvinul-A loaded-microgel particles and dialysate medium is reached. Noteworthy, at pH 6 the release amount is higher than that at pH 4.5. The reason could be the hydrophobic character of Uvinul-A. At pH 4.5 the microgel is collapsed being more hydrophobic and therefore, Uvinul-A would prefer to be inside the microgel instead of going out to the buffered medium. In contrast at pH 6, the microgel is swollen (being hydrophilic) enhancing Uvinul-A release.

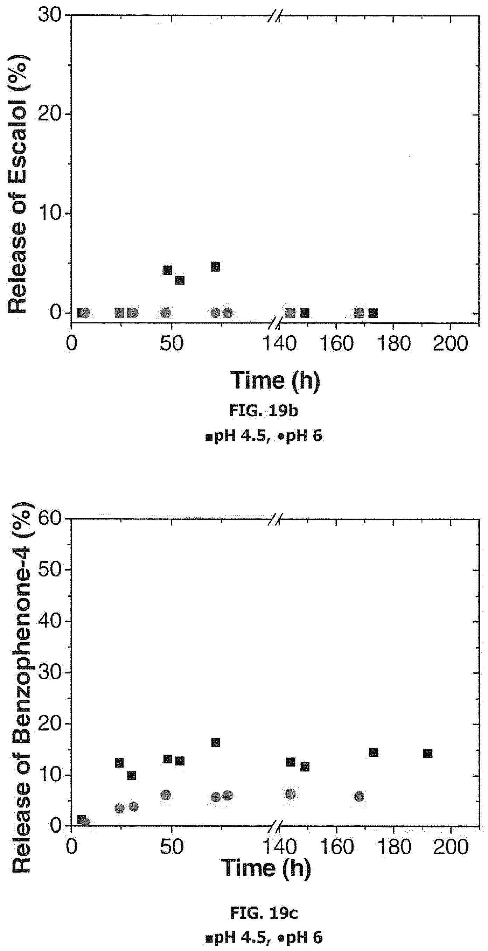

On the other hand, in the case of Escalol (Figure 19b) the release values are under 5 %. The reason could be the strong interactions between the active molecule and the microgel (that was confirmed by DOSY-NMR).

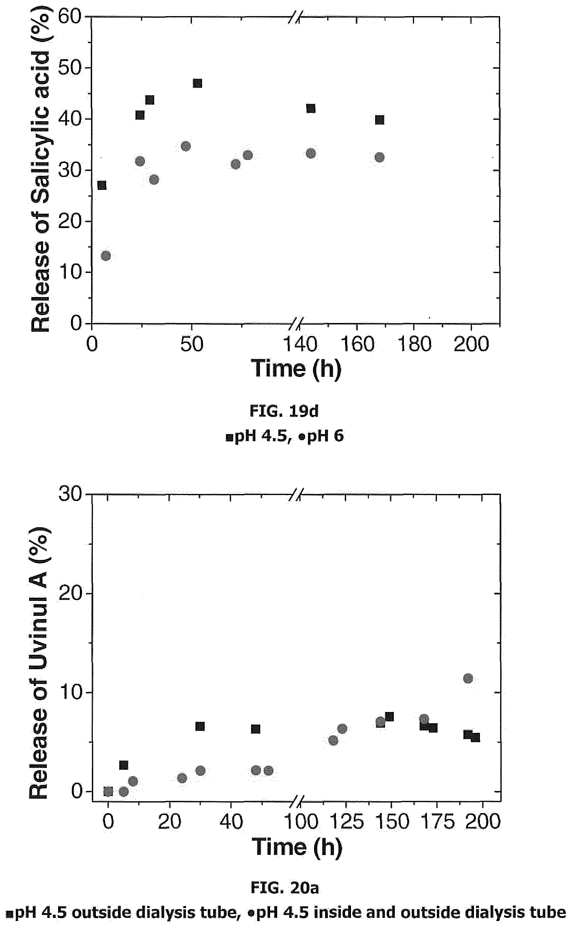

In Figure 19c and Figure 19d, the *in vitro* release of hydrophilic active molecules (Benzophenone-4 and Salicylic Acid) as a function of pH is shown. At pH 4.5 the release percentages of release is higher than those at pH 6 for both active molecules. At pH 4.5 the microgel tend to be hydrophobic meaning that there is less affinity between hydrophilic active molecules and microgel particles, so that Benzophenone-4 and Salicylic Acid prefer to move outside microgel particles (which explains the highest values of release, compared to those obtained in the case of pH 6). In addition at pH 6 microgel particles are negatively charged (deprotonation of carboxylic groups). At pH 6, Benzophenone-4 and Salicylic Acid are also negatively charged and thus, this could lead in an electrostatic repulsion between the active molecules and microgel particles. However, it seems that there is no electrostatic repulsion between microgel particles and hydrophilic active molecules. Therefore, these results suggest that hydrophobic/hydrophilic interactions are predominant in release kinetics.

b) As discussed previously, the in vitro release of different cosmetic active molecules was studied placing dialysis tubes into different dialysate buffered media (outside the dialyse tube). However, the pH of loaded-microgel dispersion was not varied. Therefore, the next step was to vary also the pH of loaded-microgel particles (dispersion placed into the dialysis tube). For that, after the encapsulation of different active molecules at a concentration of 1 mg/(mg microgel) the pH of loaded-microgel dispersion was adjusted to 4.5 using HCI and NaOH solutions.

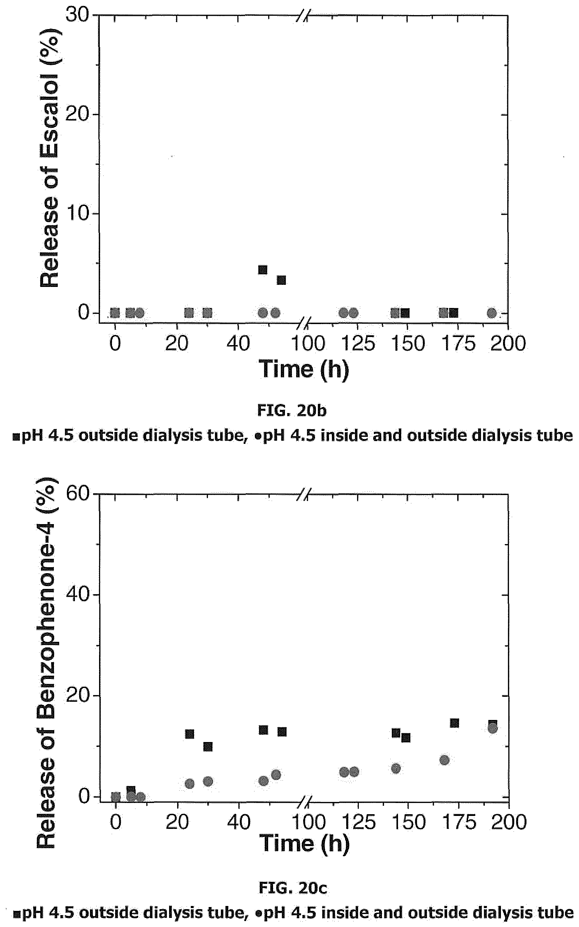

In Figures 20a and 20b, the in vitro hydrophobic active molecules (Uvinul-A and Escalol) release with time is shown. As can be seen, the release values are too low, in all the cases. In addition, no difference is observed controlling the pH of loaded-microgel particles maybe because at pH 4.5 microgel particles are collapsed.

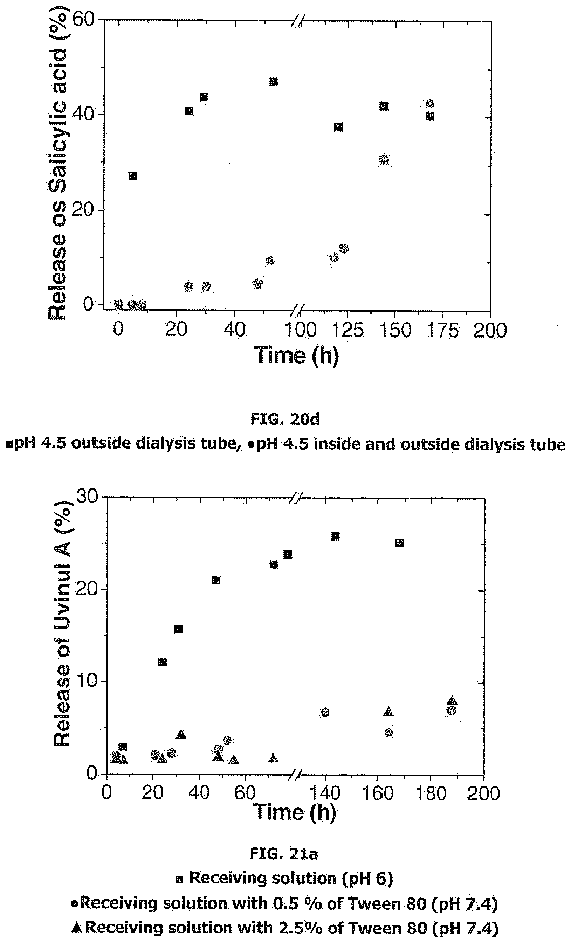

In Figures 20c and 20d, the in vitro release of hydrophilic active molecules (benzophenone-4 and salicylic acid) with time is shown. As can be observed, after adjusting the pH of loaded-microgel dispersion the released kinetics of active molecules is slower.

c) The effect of pH dialysate media composition on four active molecule release profile was studied again in other conditions.

Different dialysate medium solutions were used at 25°C: i) buffered pH 6, ii) buffered pH 7.4, iii) 0.5% Tween 80 and buffered pH 7.4, and iv) 2.5% Tween 80 and buffered pH 7.4. A mixture of 0.1% sodium azide and PBS buffer was used for pH 7.4 (at this pH, loaded-microgel particles are swollen).

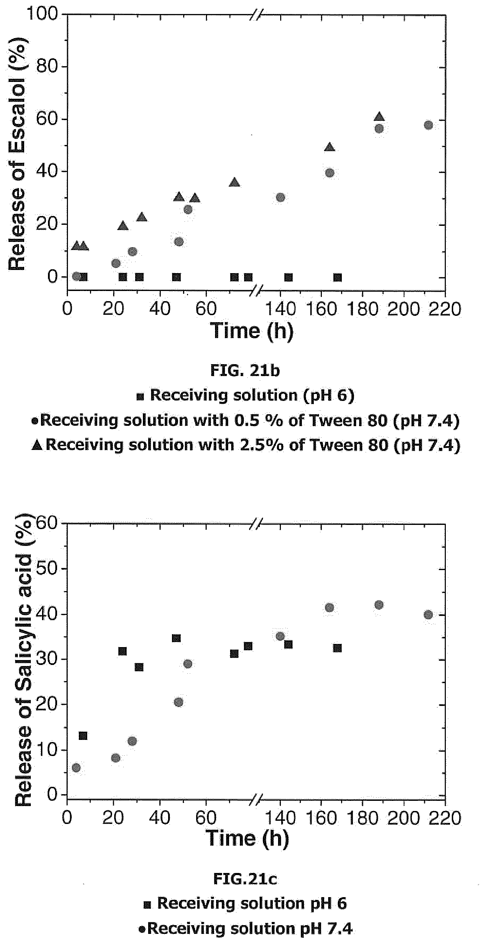

Released amount of Uvinul-A is the highest for pH 6 in the dialysate medium. The reason could be the low solubility of Uvinul-A in this medium provoking its precipitation and therefore, the lower release from dialysis tube. In the case of Escalol, although it is not soluble in the receiving solution a continuous and almost a complete release is obtained. In addition, the more concentration of Tween 80 increases, the faster the release of Escalol is. It seems that Tween 80 enhances the release of Escalol from microgel particles (see Figures 21a and 21b).

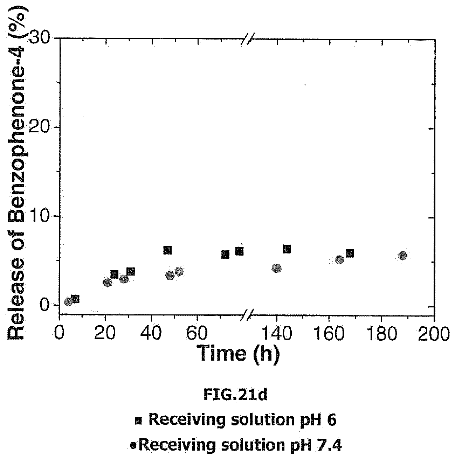

In the case of hydrophilic active molecules (benzophenone-4 and salicylic acid) there is no difference between pH 6 release kinetics and pH 7.4 release kinetics. However, the complete release is not obtained using the 'receiving solution'. Therefore, the main conclusion of this part is that the complete release of all cosmetic active molecules is obtained using a water/ethanol mixture as a release medium (see Figures 21c and 21d).

## Claims

1. Microgels for pH-triggered, temperature-triggered and/or solvent-triggered delivery of a cosmetic active organic substance, which substance is entrapped in a at least one crosslinked poly(ethylene glycol) methyl ether methacrylate polymer,
- wherein the weight ratio of cosmetic active organic substance to crosslinked polymer in the microgel is from 500 microgram/mg to 10 mg/mg, and
- wherein the crosslinked polymer comprises copolymer chains having diethylene glycol methacrylate monomeric units, oligoethylene glycol methacrylate monomeric units comprising from 6 to 10 ethylene glycol moieties, methacrylic acid monomeric units, said copolymer chains being linked with crosslinks each having di(meth)acrylate end groups and a moiety selected in the group consisting of -(CH₂-CH₂-O)ₙ-CH₂-CH₂- where n is from 0 to 6 and preferably from 4 to 5, -NH-CH₂-NH- and mixtures thereof.

2. Microgels according to claim 1, wherein the molar fraction of diethylene glycol methacrylate monomeric units is from 80 mol.% to 90 mol.%, preferably from 82 mol.% to 86 mol.%, the molar fraction of oligoethylene glycol methacrylate monomeric units is from 5 mol.% to 15 mol.%, preferably from 7 mol.% to 11 mol.%, the molar fraction of (meth)acrylic acid monomeric units is from 2 mol.% to 8 mol.%, preferably from 3 mol.% to 7 mol.%, and the molar fraction of the crosslinks is from 1 to 3 mol.%, molar fractions being the molar fractions in the crosslinked polymer.

3. Microgels according to claim 1 or claim 2, being in the form of an aqueous dispersion.

4. Microgels according to claim 1 or claim 2, being in the form of a film having a thickness being from 10 to 500 microns.

5. Microgels according to any one of the preceding claims, wherein the weight ratio of cosmetic active organic substance to crosslinked polymer is lower than 10 mg/mg and higher than a lower limit selected in the group consisting of 550 microgram/mg, 600 microgram/mg, 650 microgram/mg, 700 microgram/mg, 750 microgram/mg, 800 microgram/mg, 850 microgram/mg, 900 microgram/mg and 1 mg/mg.

6. Microgels according to any one of the preceding claims, wherein the cosmetic active organic substance is selected from the group consisting of octyl salicylate, hyaluronic acid, diethylamino hydroxybenzoyl hexyl benzoate, benzophenone-4, citronellol and salicylic acid.

7. A cosmetic composition comprising microgels according to any one of the preceding claims, wherein the composition is in the form of a make-up product, a skin care product or a UV protecting product, and wherein the composition comprises a compound selected from the group consisting of oils, surfactants, pigments and dyes, said composition having a ionic strength being from 1 mM to 20 mM.

8. Cosmetic non-therapeutic treatment method comprising a step of applying on skin, nails, lips or hair of a person, microgels according to any one of claims 1 to 6 or a cosmetic composition according to claim 7.

9. Process for the preparation of microgels according to claim 3, said process comprising a step of preparing a feeding solution of the cosmetic active organic substance in a solvent, a step of preparing an aqueous dispersion of unloaded microgel particles, a step of mixing the solution and the aqueous dispersion under stirring so as to entrap the substance into the unloaded microgel particles, and a step of recovering the microgels.

10. Process for the preparation of microgels according to claim 4, said process comprising the steps of preparing a feeding solution of the cosmetic active organic substance in a solvent, a step of preparing a film of unloaded microgel particles, a step of immersing the film in the feeding solution so as to cause swelling of the film and diffusion of the active substance into the film, and a step of recovering the microgels.

## Patentansprüche

1. Mikrogele zur pH-ausgelösten, Temperatur-ausgelösten und/oder Lösungsmittel- ausgelösten Abgabe einem kosmetischen aktiven organischen Stoff, wobei dieser Stoff in mindestens einem vernetzten Poly(ethylenglykol)methylethermethacrylat-Polymer eingeschlossen ist,
- wobei das Gewichtsverhältnis von kosmetischem aktivem organischem Stoff zu vernetztem Polymer im Mikrogel 500 Mikrogramm / mg bis 10 mg / mg beträgt, und
- wobei das vernetzte Polymer Copolymerketten mit Diethylenglykolmethacrylat-Monomereinheiten, Oligoethylenglykolmethacrylat-Monomereinheiten umfassend 6 bis 10 Ethylenglykoleinheiten, Methacrylsäuremonomereinheiten, umfasst, wobei die Copolymerketten mit Vernetzungen verbunden sind, die jeweils Di(meth)acrylat-Endgruppen und eine Einheit ausgewählt in der Gruppe bestehend aus -(CH₂-CH₂-O)ₙ-CH₂-CH₂- wobei n 0 bis 6 und vorzugsweise 4 bis 5 ist, -NH-CH₂-NH-, und Gemische davon, aufweisen.

2. Mikrogele nach Anspruch 1, wobei der Molenbruch von Diethylenglykolmethacrylat-Monomereinheiten 80 Mol-% bis 90 Mol-%, vorzugsweise 82 Mol-% bis 86 Mol-% beträgt, der Molenbruch von Oligoethylenglycolmethacrylat-Monomereinheiten 5 Mol-% bis 15 Mol-%, vorzugsweise 7 Mol-% bis 11 Mol -% beträgt, der Molenbruch der (Meth)acrylsäure-Monomereinheiten 2 Mol-% bis 8 Mol-%, vorzugsweise von 3 Mol-% bis 7 Mol-% beträgt, und der Molenbruch der Vernetzungen 1 bis 3 Mol-% beträgt, wobei Molenbrüche die Molenbrüche im vernetzten Polymer sind.

3. Mikrogele nach Anspruch 1 oder Anspruch 2 in Form einer wässrigen Dispersion.

4. Mikrogele nach Anspruch 1 oder Anspruch 2 in Form einer Folie mit einem Dicken von 10 bis 500 Mikrometern.

5. Mikrogele nach einem der vorhergehenden Ansprüche, wobei das Gewichtsverhältnis von kosmetischem aktivem organischem Stoff zu vernetztem Polymer niedriger als 10 mg / mg und höher als eine Untergrenze aus der Gruppe bestehend aus 550 Mikrogramm/mg, 600 Mikrogramm/mg, 650 Mikrogramm/mg, 700 Mikrogramm/mg, 750 Mikrogramm/mg, 800 Mikrogramm/mg, 850 Mikrogramm/mg, 900 Mikrogramm/mg und 1 mg/mg, ausgewählt wird.

6. Mikrogele nach einem der vorhergehenden Ansprüche, wobei der kosmetische aktive organische Stoff aus der Gruppe bestehend aus Octylsalicylat, Hyaluronsäure, Diethylaminohydroxybenzoylhexylbenzoat, Benzophenon-4, Citronellol und Salicylsäure, ausgewählt wird.

7. Kosmetische Zusammensetzung, umfassend Mikrogele nach einem der vorhergehenden Ansprüche, wobei die Zusammensetzung in Form eines Make-up-Produkts, eines Hautpflegeprodukts oder eines UV-Schutzprodukts vorliegt, und wobei die Zusammensetzung eine Verbindung ausgewählt aus der Gruppe bestehend aus Ölen, Tensiden, Pigmenten und Farbstoffen umfasst, wobei die Zusammensetzung eine Ionenstärke von 1 m bis 20 mM aufweist.

8. Kosmetisches nicht-therapeutisches Behandlungsverfahren, umfassend einen Schritt des Aufbringens von Mikrogelen nach einem der Ansprüche 1 bis 6 oder einer kosmetischen Zusammensetzung nach Anspruch 7 auf Haut, Nägel, Lippen oder Haare einer Person.

9. Verfahren zur Herstellung von Mikrogelen nach Anspruch 3, wobei das Verfahren einen Schritt zum Herstellen einer Beschickungslösung des kosmetischen aktiven organischen Stoffes in einem Lösungsmittel, einen Schritt zum Herstellen einer wässrigen Dispersion von unbeladenen Mikrogelpartikeln, einen Schritt zum Mischen des Lösung und die wässrige Dispersion unter Rühren, um der Stoff in die unbeladenen Mikrogelpartikel einzuschließen, und ein Schritt zur Gewinnung der Mikrogele, umfasst.

10. Verfahren zur Herstellung von Mikrogelen nach Anspruch 4, wobei das Verfahren die Schritte zur Herstellung einer Beschickungslösung der kosmetischen aktiven organischen Stoff in einem Lösungsmittel, einen Schritt zur Herstellung eines Films aus unbeladenen Mikrogelpartikeln, einen Schritt zum Eintauchen des Films in der Beschickungslösung um ein Quellen des Films und eine Diffusion des Stoffs in den Film zu verursachen, und einen Schritt des Wiederherstellens der Mikrogele, umfasst.

## Revendications

1. Microgels pour l'administration d'une substance cosmétique organique active déclenchée par le pH, la température et/ou un solvant, laquelle substance est piégée dans au moins un polymère de méthacrylate de poly (éthylène glycol) méthyl éther réticulé,
- dans lequel le rapport pondéral de la substance organique active cosmétique au polymère réticulé dans le microgel va de 500 microgrammes/mg à 10 mg/mg, et
- dans lequel le polymère réticulé comprend des chaînes de copolymère ayant des motifs monomères de méthacrylate de diéthylène glycol, des motifs monomères de méthacrylate d'oligoéthylène glycol comprenant de 6 à 10 groupements éthylène glycol, des motifs monomères d'acide méthacrylique, lesdites chaînes de copolymère étant liées par des réticulations ayant chacune des groupes terminaux di(méth)acrylate et un fragment choisi dans le groupe constitué par - (CH2-CH2-O) n-CH2-CH2- où n va de 0 à 6 et de préférence de 4 à 5, -NH-CH2-NH- et leurs mélanges.

2. Microgels selon la revendication 1, dans lesquels la fraction molaire d'unités monomères de méthacrylate de diéthylène glycol va de 80 mol% à 90 mol%, de préférence de 82 mol% à 86 mol%, la fraction molaire d'unités monomères de méthacrylate d'oligoéthylène glycol va de 5% en mole à 15% en mole, de préférence de 7% en mole à 11% en mole, la fraction molaire d'unités monomères d'acide (méth)acrylique va de 2% en mole à 8% en mole, de préférence de 2% en mole à 8% en mole, de préférence de 3% en mole à 7% en mole, et la fraction molaire des réticulations va de 1 à 3% en mole, les fractions molaires étant les fractions molaires dans le polymère réticulé.

3. Microgels selon la revendication 1 ou la revendication 2, se présentant sous la forme d'une dispersion aqueuse.

4. Microgels selon la revendication 1 ou la revendication 2, se présentant sous la forme d'un film d'épaisseur allant de 10 à 500 microns.

5. Microgels selon l'une quelconque des revendications précédentes, dans lesquels le rapport pondéral de la substance organique active cosmétique au polymère réticulé est inférieur à 10 mg/mg et supérieur à une limite inférieure choisie dans le groupe constitué de 550 microgrammes/mg, 600 microgrammes/ mg, 650 microgrammes/mg, 700 microgramme /mg, 750 microgrammes/mg, 800 microgrammes/mg, 850 microgrammes/mg, 900 microgrammes/mg et 1 mg/mg.

6. Microgels selon l'une quelconque des revendications précédentes, dans lesquels la substance organique active cosmétique est choisie dans le groupe constitué par l'octyl salicylate, l'acide hyaluronique, le diéthylamino hydroxybenzoyl hexyl benzoate, la benzophénone-4, le citronellol et l'acide salicylique.

7. Composition cosmétique comprenant des microgels selon l'une quelconque des revendications précédentes, dans laquelle la composition se présente sous la forme d'un produit de maquillage, d'un produit de soin de la peau ou d'un produit de protection UV, et dans laquelle la composition comprend un composé choisi parmi le groupe constitué d'huiles, de tensioactifs, de pigments et de colorants, ladite composition ayant une force ionique allant de 1 mM à 20 mM.

8. Procédé de traitement cosmétique non thérapeutique comprenant une étape d'application sur la peau, les ongles, les lèvres ou les cheveux d'une personne, des microgels selon l'une quelconque des revendications 1 à 6 ou d'une composition cosmétique selon la revendication 7.

9. Procédé de préparation de microgels selon la revendication 3, ledit procédé comprenant une étape de préparation d'une solution d'alimentation de la substance organique active cosmétique dans un solvant, une étape de préparation d'une dispersion aqueuse de particules de microgel non chargées, une étape de mélange de la solution et de la dispersion aqueuse sous agitation pour piéger la substance dans les particules de microgel non chargées, et une étape de récupération des microgels.

10. Procédé de préparation de microgels selon la revendication 4, ledit procédé comprenant les étapes de préparation d'une solution d'alimentation de la substance organique active cosmétique dans un solvant, une étape de préparation d'un film de particules de microgel non chargées, une étape d'immersion du film dans la solution d'alimentation de manière à provoquer un gonflement du film et la diffusion de la substance active dans le film, et une étape de récupération des microgels.
